(19) **Europäisches Patentamt**
**European Patent Office**
**Office européen des brevets**

(11) Publication number: **0 222 580**
**A1**

(12) # EUROPEAN PATENT APPLICATION

(21) Application number: **86308565.0**

(22) Date of filing: **03.11.86**

(51) Int. Cl.⁴: **A 61 K 7/32**

(30) Priority: **04.11.85 US 794981**

(43) Date of publication of application:
**20.05.87 Bulletin 87/21**

(84) Designated Contracting States:
**AT BE CH DE FR GB IT LI LU NL SE**

(71) Applicant: **CALGON CORPORATION**
**Route 60-Campbell's Run Road**
**Robinson Township Pennsylvania 15205 (US)**

(72) Inventor: **Klein, William L.**
**454 Chestnut Street**
**Nutley New Jersey 07110 (US)**

**Sykes, Arthur R.**
**23 Brooktree Road**
**East Windsor New Jersey 08520 (US)**

(74) Representative: **Crampton, Keith John Allen et al**
**D YOUNG & CO 10 Staple Inn**
**London WC1V 7RD (GB)**

(54) **Antiperspirant formulations containing quaternary ammonium polymer.**

(57) Stable antiperspirant compositions comprise an antiperspirant and a polymer of dimethyldiallyl ammonium chloride, which serves to increase the residual amount of the antiperspirant on a surface such as the skin.

EP 0 222 580 A1

Bundesdruckerei Berlin

## Description

### ANTIPERSPIRANT FORMULATIONS CONTAINING QUATERNARY AMMONIUM POLYMER

This invention relates to antiperspirant compositions. Numerous literature references disclose detergent compositions for cosmetic and personal use, such as shampoos, antiperspirant formulations and anti-dandruff rinse conditioners that contain anionic and/or cationic polymers, an active agent, surfactants, emollients, and other additives and preservatives common in the industry, as discussed below.

U.S. Patent Specification US-A-3 761 417 is directed to detergent compositions containing particle-deposition-enhancing agents, more specifically, toilet detergent bars containing water-insoluble particles such as antimicrobial agents, organic surfactants and cationic polymers. Surfactants are an essential ingredient of the compositions. Dimethyldiallyl ammonium chloride (DMDAAC) is specifically mentioned as a possible cationic polymer.

US. Patent Specification US-A-3 769 398 discloses non-anionic hair shampoo formulations containing an active ingredient (a detergent such as a betaine, sulfobetaine or amine oxide or a mixture thereof), a water-soluble polymer (polyethylenimine-ethylene oxide or propylene oxide) and propoxylated polyethylenimines.

U.S. Patent Specification US-A-4 329 335 describes an amphotericnonionic anti-dandruff shampoo containing 1-imidazolyl-1-(4-chlorophenoxy)-3,3-dimethylbutan-2-one and amphoteric surfactants polyoxyethylene hexitan mono or higher fatty acid ester, tertiary amine oxide, fatty acid mono- or di-ethanolamide and optionally a polymerized quarternized ammonium compound. DMDAAC is specifically disclosed as a preferred polymerized quarternized ammonium compound.

European Patent Specification EP-A-0 074 819 discloses an anti-dandruff cream rinse conditioner of DMDAAC-acrylamide, glucan or guar gum and hydroxyethylcellulose.

European Patent Specification EP-A-0 055 857 discloses the preparation of topically applied compositions comprising ultraviolet-light-absorbing materials and film-forming polymers and exhibiting enhanced protection from erythema-causing radiation.

It has now been found that stable and effective antiperspirant compositions can be prepared comprising at least one antiperspirant and one or more polymers of dimethyldiallyl ammonium chloride, and that the polymer increases the amount of antiperspirant on the surface to which it is applied, thereby enhancing its effect and making a lower dosage of the antiperspirant effective, thereby reducing possible future side effects.

The present invention provides an antiperspirant composition comprising at least one antiperspirant agent and a polymer of dimethyldiallyl ammonium chloride.

Also in accordance with the invention, body secretion is treated by topically applying such a composition to the skin.

The compositions of the present invention are thus capable of being topically administered. They may optionally contain cosmetically acceptable excipients such as buffers and/or preservatives. The polymer is present in an amount sufficient to enhance deposition and/or retention of the antiperspirant agent on the intended surface. The composition may be prepared by agitating the polymer, with or without de-ionized water, otpionally adding an emulsifier with continued agitation, heating to a temperature ranging from ambient to 90°C (preferably 65° to 80°) until a uniform mixture is obtained, and continuing agitation while adding an antiperspirant agent, optionally followed by preservatives, thickening agents, buffers, dyes and/or fragrances, and ultimately adjusting the formulation by adding de-ionized water while maintaining the temperature. The polymer can be added at any stage of the procedure.

The amount of the antiperspirant agent varies over a wide range and depends on the specific agent involved. Usually, the antiperspirant effective amount would be considerably less than that previously believed necessary to obtain the desired results. Specifically, the amount of antiperspirant agent used in the formulations herein usually constitutes from 0.01% to 50% and preferably 5% to 20% of the total weight of the composition. If the only components are antiperspirant agents and polymers, the amount can be up to 99.09% by weight.

The amount of polymer used in the practice of the invention ranges from 0.01% to 12% polymer, preferably 1% to 3%, by weight of the composition, the remainder being the antiperspirant agent and optional conventional acceptable excipients.

Polymers of dimethyldiallyl ammonium chloride (DMDAAC) include both homopolymers and copolymers of DMDAAC. The copolymer should contain at least 10% by weight of DMDAAC. A homopolymer is preferred. An example of a homopolymer of DMDAAC is that sold under the registered trade mark Merquat 100, which is manufactured by Calgon Corporation and which has an intrinsic viscosity of 0.3 dl/g in 1.0 $\underline{M}$ NaCl. Although any copolymers (including terpolymers and polymers of more than three monomers) may be used, the preferred copolymers are DMDAAC/acrylamide and DMDAAC/acrylic acid.

Example of copolymers of dimethyldiallyl ammonium chloride (DMDAAC) and acrylamide, are Merquat S and Merquat 550.

The preferred product is Merquat S. Merquat 550 and S copolymers have an intrinsic viscosity of 4.2 ± 0.2 in 1.0 $\underline{M}$ NaCl and contain, by weight, about 50 percent DMDAAC and 50 percent acrylamide.

Although any ratio of DMDAAC and comonomer will work, the preferred ratio is 10 to 75 percent, particularly 25 to 75 percent, by weight, DMDAAC and, correspondingly, 25 to 90 percent, particularly 25 to 75 percent, by weight, comonomer.

Polymers of DMDAAC used in the invention have a molecular weight ranging from 20,000 to 2,500,000, preferably from 200,000 to 300,000, and an intrinsic viscosity of 0.1 to 2.5, preferably from 0.7-0.9, in 1.0M NaCl.

Representative antiperspirant agents used in the practice of the invention are aluminium zirconium complexes such as those sold under the registered Trade Marks Rezal 36GP (aluminium zirconium tetrachlorohydrex-glycine) and Rezal 67 (aluminium zirconium pentachlorohydrate), aluminium chlorohydrol, aluminium chlorohydroxide, aluminium chlorohydrate, aluminium chlorohydrex P.G., aluminium sulphate and potassium alum.

Representative emollients, emulsifiers, humectants and moisturising agents that can be used in the compositions of the invention are $C_{12-15}$alcohol benzoates, SDA40 alcohol, stearyl alcohol, sorbitol, Volpo 3100, glycerin, glyceryl stearate, propylene glycol (PEG), lanolin, talc, vegetable oils, mineral oils, isopropyl myristate, aloe vera, cyclomethicone, jojoba oil, PEG 100 stearate, cetyl esters, PPG-15 stearyl ether and steareth 2 and 21.

Other cosmetically acceptable excipients that the formulation may contain are thickening agents, buffering agents and preservatives. Suitable water soluble preservatives are sodium bisulphite, sodium thiosulphate, ascorbates, benzalkonium chloride, glydant, 1,3-bis(hydroxymethyl)-5,5-dimethyl-2,4-imidazolidinedione, chlorobutanol, thimerosal, phenylmercuric borate, Dowicil 200, 1-(3-chloro-2-propenyl)-3,5,7-triaza-1-azoniatricyclo[3.3.1.1]decane, parabens, Tektamer 38 (1,2-dibromo-2,4-dicyanobutane), benzyl alcohol and phenylethanol. Suitable thickening agents are those sold under the registered Trade Marks Cab-O-Sil M5, Carbomer 934, 940 and 941, sodium stearate, magnesium aluminium silicate and hydroxyethyl cellulose. These agents, may optionally be present in amounts of from 0.05 to 50% by weight, preferably 1 to 5%. Suitable watersoluble buffering agents include alkali or alkaline-earth metal carbonates, phosphates, bicarbonates, citrates, borates, acetates, acid anhydrides and succinates, such as sodium phosphate, citrate, borate, acetate, bicarbonate and carbonate. These agents may be present in amounts sufficient to maintain the optimum pH of the system in the range 2 to 9. As such the buffering agent can be as much as 20% on a weight to weight basis of the total composition.

Studies were conducted on the degree of retention of aluminium on the skin with and without a polymer.

## TABLE I

### RETENTION OF ALUMINUM ON SKIN

| Samples | Treatment | Results (PPM) |
|---|---|---|
| A. Control | Skin sample treated with 150 µl of aluminum chlorohydrate P.G. antiperspirant solution followed by 15-second tap water rinse | 0.4* |
| B. Experiment | Skin sample treated with 150 µl of aluminum chlorohydrate P.G. antiperspirant solution with 2% Merquat 100 followed by 15-second tap water rinse | 0.8* |

*same results for two (2) experiments

The above results clearly illustrate that formulation B which contains a polymer is twice as effective as formulation A without a polymer in the retention of aluminium on the skin.

The following examples illustrate the preparation of various antiperspirant formulations of the invention. The Examples do not limit the invention.

4

# EXAMPLE 1

## Stick Antiperspirant Control Formulation*

| NO. | PHASE | INGREDIENT | % BY WEIGHT |
|-----|-------|-----------|-------------|
| 1 | A | cyclomethicone | 46.20 |
| 2 | A | glyceryl stearate and PEG 100 stearate | 1.00 |
| 3 | B | stearyl Alcohol | 20.00 |
| 4 | B | Volpo 3100 | 10.00 |
| 5 | B | Rezal 36GP | 20.00 |
| 6 | C | talc | 1.00 |
| 7 | D | triclosan | 0.20 |
| 8 | D | Cab-O-Sil M5 | 1.00 |
| 9 | E | fragrance | 0.60 |
| | | q.s. | 100.00 |

*Without polymer

With rapid agitation, phase A ingredients were thoroughly mixed and heated to 65°C while phase B ingredients were individually added and stirred until dissolved. The solution was stirred for approximately 5 minutes while adding phase C. When dissolved, the resulting phases D were added individually and stirred for 15 minutes and phase E was added, agitated until dissolved and q.s. to 100%. The resulting mixture was then poured into the suitable containers for personal care application. The temperature was maintained at 65°C while making the additions.

## EXAMPLE 2

### Stick Antiperspirant

| NO. | PHASE | INGREDIENT | % BY WEIGHT |
|-----|-------|------------|-------------|
| 1 | A | cyclomethicone | 44.20 |
| 2 | A | glyceryl stearate and PEG 100 stearate | 1.00 |
| 3 | B | stearyl Alcohol | 20.00 |
| 4 | B | Volpo 3100 | 10.00 |
| 5 | B | Rezal 36GP | 20.00 |
| 6 | C | talc | 1.00 |
| 7 | D | triclosan | 0.20 |
| 8 | D | Cab-O-Sil M5 | 1.00 |
| 9 | E | perfume | 0.60 |
| 10 | E | Merquat 100 | 2.00 |
| | | | 100.00 |

The procedure and reaction conditions of Example 1 were followed except that "Merquat 100" was added with phase E ingredients to obtain the corresponding product.

## EXAMPLE 3

### Stick Antiperspirant

| NO. | PHASE | INGREDIENT | % BY WEIGHT |
|-----|-------|-----------|-------------|
| 1 | A | cyclomethicone | 43.20 |
| 2 | A | glyceryl stearate and PEG 100 stearate | 1.00 |
| 3 | B | stearyl Alcohol | 20.00 |
| 4 | B | Volpo 3100 | 10.00 |
| 5 | B | Rezal 36GP | 20.00 |
| 6 | C | talc | 1.00 |
| 7 | D | triclosan | 0.20 |
| 8 | D | Cab-O-Sil M5 | 1.00 |
| 9 | E | fragrance | 0.60 |
| 10 | E | Merquat S | 3.00 |
|  |  |  | 100.00 |

With rapid agitation, phase A ingredients were thoroughly mixed and heated to 65°C. Phase B ingredients were individually added to phase A and stirred for 5 minutes. Phase C ingredient was added and stirred for 5 minutes. Phase D ingredients were individually added and stirred for 15 minutes. With continued agitation, phase E ingredients were added individually and stirred until dissolved. The resulting mixture was then stirred an additional 15 minutes. This mixture was then poured into the suitable containers for personal care application. The temperature was maintained at 65°C while making the additions.

Following the above reaction conditions and procedure, aluminium chlorohydrol, aluminium sulphate, potassium alum, aluminium chlorohydrate, or aluminium chlorohydrex P.G. may be substituted for aluminium zirconium tetrachlorohydrex complex, to obtain the corresponding stick antiperspirant formulation.

## EXAMPLE 4

## Roll-on Antiperspirant

| NO. | PHASE | INGREDIENT | % BY WEIGHT |
|-----|-------|------------|-------------|
| 1 | A | cyclomethicone | 44.20 |
| 2 | A | glyceryl stearate and PEG 100 stearate | 1.00 |
| 3 | B | Merquat 100 | 2.00 |
| 4 | B | stearyl alcohol | 20.00 |
| 5 | B | silica | 1.00 |
| 6 | B | Volpo 3100 | 10.00 |
| 7 | C | aluminum zirconium tetra-chlorohydrex complex | 20.00 |
| 8 | C | talc | 1.00 |
| 9 | D | preservative & fragrance q.s. | qs 100.00 |

In separate vessels, the ingredients of phases A and B were heated to 65°C. With agitation, phase B was added to phase A, cooled to 65°C and phase C ingredients were added. Agitation was continued and the reaction mixture was cooled and phase D ingredient added.

## EXAMPLE 5

### Antiperspirant Cream

| NO. | PHASE | INGREDIENT | % BY WEIGHT |
|-----|-------|------------|-------------|
| 1 | A | cetyl alcohol | 4.00 |
| 2 | A | glyceryl stearate and PEG 100 stearate | 10.00 |
| 3 | B | Merquat 100 | 2.00 |
| 4 | B | sorbitol | 3.00 |
| 5 | B | deionized water | 41.00 |
| 6 | C | aluminum zirconium tetra-chlorohydrex complex | 40.00 |
| 7 | D | preservative & fragrance q.s. | 100.00 |

In separate vessels, the ingredients of phases A and B were heated to 75°C and 78°C, respectively. With agitation, phase B was added to phase A, cooled to 40°C and phase C ingredients were added. Agitation was continued and the reaction mixture was cooled and poured into suitable containers for personal care application.

## EXAMPLE 6

### Liquid Antiperspirant

| NO. | PHASE | INGREDIENT | % BY WEIGHT |
|-----|-------|------------|-------------|
| 1 | A | SDA 40 alcohol | 25.00 |
| 2 | A | glycerine | 4.00 |
| 3 | B | aluminium chlorohydrate | 30.00 |
| 4 | C | deionized water | 35.80 |
| 5 | D | Merquat 100 | 2.00 |
| 6 | E | preservative & fragrance q.s. | 100.00 |

In the same vessels, the ingredients of phases A and B were added and mixed until a clear solution was obtained. With agitation, phases C and D were premixed and added to the reaction mixture. Agitation was continued until the reaction mixture was uniform, phase D ingredient added and poured into suitable containers for personal care application.

## EXAMPLE 7

## Antiperspirant Lotion

| NO. | PHASE | INGREDIENT | % BY WEIGHT |
|-----|-------|------------|-------------|
| 1 | A | PPG-15 stearyl ether | 4.00 |
| 2 | A | steareth 21 | .60 |
| 3 | A | steareth 2 | 2.60 |
| 4 | B | deionized water | 50.80 |
| 5 | B | Merquat 100 | 2.00 |
| 6 | C | aluminum zirconium tetra-chlorohydrex-glycine | 40.00 |
| 7 | D | preservative & fragrance q.s. | 100.00 |

In separate vessels, the ingredients of phases A and B were heated to 75°C and 78°C, respectively. With agitation, phase B was added to phase A, cooled to 40°C and phase C ingredient was added. Agitation was continued, phase D ingredient was added and the reaction mixture was cooled and poured into suitable containers for personal care application.

## EXAMPLE 8

### Stick Antiperspirant

| NO. | PHASE | INGREDIENT | % BY WEIGHT |
|-----|-------|------------|-------------|
| 1 | A | isopropyl myristate | 2.00 |
| 2 | A | propylene glycol | 20.30 |
| 3 | B | propylene glycol | 2.00 |
| 4 | B | titanium dioxide | 0.20 |
| 5 | C | isostearyl alcohol | 11.30 |
| 6 | D | stearamide MEA | 26.00 |
| 7 | E | SDA 40 alcohol | 14.50 |
| 8 | E | aluminium chlorohydrex P.G. | 20.00 |
| 9 | F | triclosan | 0.20 |
| 10 | G | Merquat S | 3.00 |
| 11 | H | preservatives and fragrances q.s. | 100.00 |

With rapid agitation, phase A ingredients were thoroughly mixed and heated to 65°C. Phase B ingredients were thoroughly mixed added to phase A with continued agitation. Phase C ingredient was added stirred for 5 minutes and the reaction mixture heated to 80°C. Phase D ingredient was added and stirred to obtain uniformity. With continued agitation, the reaction was cooled to 76°C and phase E ingredients were added individually and stirred until dissolved. The resulting mixture was then briefly stirred and phases F, G and H were successively added. This mixture was then poured into the suitable containers for personal care application.

**Claims**

1. An antiperspirant composition comprising at least one antiperspirant agent and a polymer of dimethyldiallyl ammonium chloride.

2. A composition as claimed in claim 1, further comprising water.

3. A composition as claimed in claim 2, in which the antiperspirant agent is aluminium zirconium complex, aluminium sulphate, potassium alum, aluminium chlorohydrol, aluminium chlorohydroxide, aluminium chlorohydrate or aluminium chlorohydrex P.G.

4. A composition as claimed in claim 4, in which the antiperspirant agent is aluminium zirconium complex, aluminium chlorohydrex P.G. or a combination thereof.

5. A composition as claimed in claim 4, in which the antiperspirant agent is aluminium zirconium tetrachlorohydroxy-glycine and/or aluminium zirconium pentachlorohydrate.

6. A composition as claimed in any one of claims 2 to 5, in which the polymer is a homopolymer of dimethyldiallyl ammonium chloride.

7. A composition as claimed in any one of claims 2 to 5, in which the polymer is dimethyldiallyl ammonium chloride/acrylamide or dimethyldiallyl ammonium chloride/acrylic acid.

8. A composition as claimed in claim 7, in which the copolymer contains 10 to 75 percent by weight dimethyldiallyl ammonium chloride and 25 to 90 percent by weight acrylamide.

9. A composition as claimed in any one of claims 2 to 8, further comprising cosmetically acceptable excipients.

10. A composition as claimed in claim 1 for use in treating body secretion by being topically administered to the skin.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 018 137 (McLAUGHLIN GORMLEY KING CO.) * Example 22 * | 1-3,6, 9,10 | A 61 K 7/32 |
| Y | GB-A-2 004 743 (L'OREAL) * Whole document * | 1-7,9, 10 | |
| Y | GB-A-2 113 706 (COLGATE-PALMOLIVE) * Whole document * | 1-7,9, 10 | |
| Y | US-A-4 174 386 (SPITZER et al.) * Whole document * | 1-7,9, 10 | |
| | | | **TECHNICAL FIELDS SEARCHED (Int. Cl. 4)** |
| A | SOAP, PERFUMERY & COSMETICS, vol. 57, no. 7, July 1984, pages 351,355, London, GB; P. ALEXANDER: "Merquat - its qualities and applications" * Page 351, middle column, line 11 * | 1 | A 61 K |

The present search report has been drawn up for all claims

| Place of search | Date of completion of the search | Examiner |
|---|---|---|
| THE HAGUE | 22-01-1987 | FISCHER J.P. |